Europäisches Patentamt

**European Patent Office**

Office européen des brevets

(19)

(11) Numéro de publication: **0 263 906**
**B1**

(12) **FASCICULE DE BREVET EUROPEEN**

(45) Date de publication du fascicule du brevet:
**19.12.90**

(51) Int. Cl.⁵: **G01N 27/68,** G01N 33/00

(21) Numéro de dépôt: **86402333.8**

(22) Date de dépôt: **17.10.86**

(54) **Dispositif de détection et de mesure de concentrations en CO2 et CO.**

(43) Date de publication de la demande:
**20.04.88 Bulletin 88/16**

(45) Mention de la délivrance du brevet:
**19.12.90 Bulletin 90/51**

(84) Etats contractants désignés:
**DE NL**

(56) Documents cités:
**FR-A- 857 288**
**US-A- 3 973 914**
**US-A- 4 259 292**

**P.PASCAL: "NOUVEAU TRAITE DE CHIMIE MINERALE", vol. VIII, 1968, Premier Fascicule, éditeur P.Amiel et al., Masson et Cie., éditeurs, Paris, FR;**

(73) Titulaire: **P.G.E.P. PROFESSIONAL GENERAL ELECTRONIC PRODUCTS Société Anonyme, 130 rue Jean-Pierre Timbaud, F-92400 Courbevoie(FR)**

(72) Inventeur: **Goldman, Alice, Physique des Décharges Rech.No.114 du CNRS, Plateau du Moulon F-91190 Gif-Sur-Yvette(FR)**
Inventeur: **Goldman, Max, Physique des Décharges Rech.No.114 du CNRS, Plateau du Moulon F-91190 Gif-Sur-Yvette(FR)**
Inventeur: **Roos, André, Hotel du Bon Accueil, Chaffour F-78270 Bobnieres(FR)**
Inventeur: **Boukhalfa, Noureddine, Physique des Décharges Rech.No.114 du CNRS, Plateau du Moulon F-91190 Gif-Sur-Yvette(FR)**
Inventeur: **Amouroux, Jacques, Physique des Décharges Rech.No.114 du CNRS, Plateau du Moulon F-91190 Gif-Sur-Yvette(FR)**

(74) Mandataire: **Laget, Jean-Loup, Cabinet Pierre Loyer 77, rue Boissière, F-75116 Paris(FR)**

## Description

La présente invention concerne un dispositif de détection et de mesure de concentration de $CO_2$, ainsi que de CO dans le cas d'une atmosphère contenant CO et $CO_2$.

On connait de nombreux procédés pour la mesure de concentration de $CO_2$, tels que la spectroscopie ou la chromatographie; ces différents procédés connus nécessitent cependant un appareillage relativement onéreux et requièrent une certaine technicité, ce qui réserve leur usage au laboratoire, ou bien imposent l'intervention d'un technicien pour leur utilisation et l'interprétation des résultats.

On connait également par le brevet français n° 857 288 du 24 mars 1939 des dispositifs pour provoquer la dissociation d'un gaz par l'application d'une étincelle électrique entre deux électrodes métalliques. Par ailleurs, les chimistes ont déjà réalisé en laboratoire la dissociation du $CO_2$ en CO par des étincelles électriques (Nouveau traité de chimie minérale, P. Pascal, page 699).

L'un des buts de l'invention est de proposer un dispositif de détection et de mesure de concentrations de $CO_2$ et CO qui soit à la fois de faible coût, facile à installer et à exploiter, tout en permettant la mesure de faibles teneurs (quelques centaines de ppm au moins).

Un tel dispositif pourra notamment être utilisé pour le dosage du $CO_2$ de l'atmosphère ambiante (de l'ordre de 300 ppm), ou bien dans différents secteurs industriels tels que les industries agro-alimentaires, ou l'industrie automobile pour la mesure des teneurs en $CO_2$ et CO dans les gaz d'échappement d'un moteur à combustion interne.

A cet effet, selon l'invention, le dispositif de détection et de mesure de concentration de gaz comprend:

– une cellule avec une conduite d'entrée du gaz à analyser et une conduite de sortie du gaz analysé,

– à l'intérieur de cette cellule, des moyens générateurs de haute tension propres à produire une décharge dans le gaz de manière à provoquer la dissociation du gaz,

– en aval de la cellule, des moyens détecteurs,

– caractérisé en ce que, pour la détection et la mesure de la concentration de $CO_2$ et CO, une seule électrode est reliée auxdits moyens générateurs de haute tension propre à assurer la dissociation du $CO_2$ en CO, et en ce que lesdits moyens détecteurs assurent la mesure de la concentration en CO.

En effet, le CO étant plus facile à mesurer de façon simple et peu coûteuse que le $CO_2$, on dissocie le $CO_2$ par décharge et c'est sur la quantité de CO ainsi formée qu'est effectuée la mesure. Un étalonnage approprié de l'appareil permet une lecture directe de la teneur en $CO_2$ du gaz analysé, et une mesure préalable donne la teneur du gaz en CO.

Le type de décharge dépendra de différents paramètres, notamment de la concentration du $CO_2$ à mesurer.

Il est possible de prévoir :

. soit une décharge couronne, pour les concentrations relativement élevées, qui permettra de produire une dissociation avec une très faible consommation d'énergie.

. soit une décharge en régime d'arc, qui permettra la mesure de très faibles concentrations (plus faibles que la concentration atmosphérique naturelle), grâce à une dissociation plus importante, mais au prix d'une consommation d'énergie plus élevée.

Très avantageusement, les moyens détecteurs comprennent un capteur disposé à l'intérieur de la cellule de mesure, notamment un capteur à semi-conducteur à effet catalytique.

On connaît en effet des capteurs de CO à semi-conducteur très fiables et de très faible coût. Dans un tel composant, les réactions catalytiques induites par le CO sur le matériau semi-conducteur entraînent une diminution de sa résistance ; il suffira donc de mesurer ce paramètre pour obtenir une indication du taux de CO, et donc indirectement du taux de $CO_2$ également.

On notera cependant que l'utilisation d'un capteur à semi-conducteur n'est pas nécessaire ; tout autre type de détecteur de faible coût pouvant être utilisé, notamment des indicateurs chimiques à virage de couleurs ; si l'on utilise des indicateurs contenus dans une colonne, la longueur de colonne ayant viré de couleur pourra donner une indication quantitative sur les concentrations en CO et $CO_2$ dans l'atmosphère à analyser.

De façon également très avantageuse, il est prévu au moins une grille d'arrêt des ions, interposée entre l'électrode et les moyens détecteurs.

Une telle grille, outre le fait qu'elle se laisse traverser par le flux de gaz sans créer de perturbation sensible dans l'écoulement de celui-ci, permet d'arrêter les ions en ne laissant passer que les particules neutres, éliminant donc un certain "bruit de fond" du détecteur en raison de la sensibilité de celui-ci aux particules chargées.

Cette grille peut être unique ; il est également possible de prévoir une pluralité de grilles placées les unes derrière les autres, polarisées progressivement de manière appropriée pour arrêter la plus grande quantité de particules chargées.

De préférence, dans le cas où le capteur est disposé à l'intérieur de la cellule de mesure, la grille d'arrêt est en forme de cage de Faraday entourant le capteur pour le rendre insensible aux parasites électriques ambiants.

La grille assure donc, outre sa fonction de grille d'arrêt, une seconde fonction de blindage du détecteur, qui rendra celui-ci insensible aussi bien aux parasites industriels ambiants qu'aux parasites électriques produits par la décharge.

Dans un mode de réalisation particulier, les conduites d'entrée et de sortie sont réalisées en forme de venturi, ce qui permet de faciliter la circulation du gaz à analyser, qui s'effectue de façon naturelle dans la cellule grâce à l'effet de "vent électrique" produit par la décharge au voisinage de la pointe (aspiration du gaz environnant en arrière de la pointe pour le refouler en avant de celle-ci, c'est-à-dire ici en direction du détecteur).

Les conduites d'entrée et de sortie en forme de venturi permettent d'assurer un écoulement le plus laminaire possible au voisinage de l'électrode et du capteur, sans perturber la décharge la forme intérieure de la cellule pourra être optimisée pour assurer un écoulement du flux de gaz dans les meilleures conditions.

En variante, le flux de gaz pourrait être soumis à une circulation forcée, l'étalonnage du dispositif étant différent dans ce cas. Il est alors souhaitable de prévoir un diamètre de cellule le plus petit possible, pour diminuer la quantité de gaz devant être forcé dans la cellule pour analyse. Dans ce cas, la cellule de décharge pourra avantageusement avoir une structure fil-cylindre, la circulation du gaz se faisant selon l'axe du système, et l'ensemble grille-détecteur étant disposé à la face de sortie du cylindre.

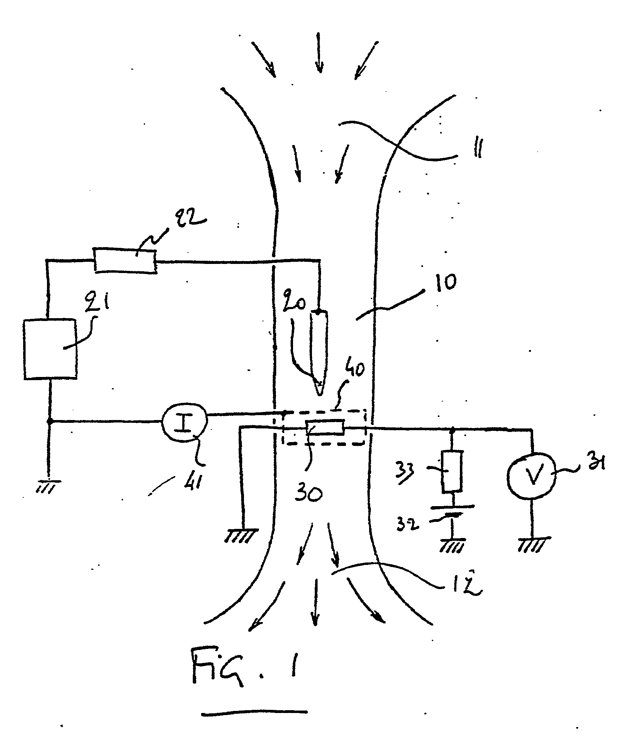

D'autres caractéristiques et avantages de l'invention apparaîtront à la lecture de la description détaillée ci-dessous d'un example de réalisation, faite en référence à la figure unique annexée, qui représente schématiquement l'ensemble des éléments du dispositif.

Sur cette figure, on a illustré le mode de réalisation à circulation naturelle, par exemple pour la mesure de la concentration de $CO_2$ et CO dans l'air ambiant.

Une cellule 10, de forme sensiblement cylindrique, comporte en partie supérieure et en partie inférieure deux ouvertures 11 et 12 évasées formant venturi.

Au centre de la cellule, en position axiale, est placée une électrode 20 reliée à des moyens générateurs de haute tension 21 par l'intermédiaire d'une résistance ballast 22.

L'électrode 20 comporte une pointe établir et entretenir la décharge, grâce à la très forte déformation du champ électrique dans cette région ("effet de pointe" classique). De préférence, la pointe est dirigée axialement vers l'ouverture 12 pour profiter de l'effet de "vent électrique" et créer un courant de fluide dans la cellule de l'ouverture 11 (amont) vers l'ouverture 12 (aval).

Un capteur 30 de mesure de concentration de CO est disposé en aval l'électrode, et relié à des moyens de mesure ; dans le cas où le paramètre variable est la résistance du capteur, on peut par example prévoir un millivoltmètre 31 placé en dérivation sur le capteur, celui-ci étant alimenté par une source de tension 32 par l'intermédiaire d'une résistance série 33.

Une grille d'arrêt 40 est interposée entre l'électrode 20 et le capteur 30 ; elle va permettre, sans arrêter ni perturber le flux de gaz, d'empêcher les ions gazeux produits par la décharge de parvenir jusqu'au capteur, ce qui risquerait de fausser les indications données par celui-ci.

Cette grille est par example reliée au potentiel de la masse par l'intermédiaire d'un milliampèremètre ou microampèremètre 41 qui donnera une indication sur le courant nécessaire à l'entretien de la décharge.

De préférence, cette grille est conformée de manière à entourer complètement le capteur 30 pour le rendre insensible aux parasites électriques ambiants par effet de cage de Faraday.

Un capteur particulièrement approprié au dispositif de l'invention est notamment le capteur TGS # 812 commercialisé par MXE Engineering b.v., qui présente une excellente sensibilité au CO, est de très faible dimension (17 mm de diamètre), très facile à mettre en oeuvre (mesure d'une résistance variable), et au surplus robuste, fiable et bon marché.

En ce qui concerne la haute tension permettant la décharge, celle-ci peut être produite par le générateur 21 selon différents modes :

. il peut s'agir tout d'abord d'une tension continue ;

. la tension peut être également alternative, ce qui évite l'emploi de redresseurs. Le rendement de dissociation n'est pas le même selon la polarité, mais l'effet obtenu reste identique, de sorte qu'un étalonnage spécifique pourra permettre de doser le $CO_2$ de la même façon que si la tension était continue ; enfin, la tension peut être simplement produits sous forme impulsionnelle, de manière à économiser l'énergie: la décharge n'est ainsi produite que lorsque l'on souhaite procéder à une mesure (par exemple une impulsion toutes les heures).

La valeur de la haute tension sera choisie en fonction du type de décharge que l'on souhaite réaliser :

. pour une décharge couronne (qu'elle soit continue, alternative, ou impulsionnelle), la haute tension pourra être comprise entre 1kV et 20 à 30 kV, le courant moyen variant entre quelques µA et quelques mA. Un tel régime, tout en ne nécessitant qu'une faible puissance (quelques W) permettra la dissociation du $CO_2$ avec un rendement suffisant pour doser des taux de quelques centaines de ppm.

Le régime de décharge couronne sera avantageusement un régime limitant la zone d'ionisation au voisinage de l'électrode haute tension, ce qui limitera la production parasite d'ozone résultant en partie de la recombinaison de l'oxygène atomique produit par la décharge avec les molécules d'oxygène de l'air ambiant et résultant de la décomposition du $CO_2$. Etant donné que les capteurs à semi-conducteur présentent souvent une certaine sensibilité à l'ozone, ceci constitue un point important qui pourra également être partiellement résolu par un choix approprié (argent ou laiton) du matériau de l'electrode haute tension, ainsi que par la mise en place d'un catalyseur de destruction de l'ozone sur la face active du détecteur. De toute façon, l'étalonnage permettra de s'affranchir de façon aisée de cet inconvénient.

. pour une décharge en régime d'arc (stable ou coupé) la haute tension n'interviendra que pour l'amorçage de l'arc, la tension nécessaire pour le maintien de l'arc une fois amorcé étant très faible (de l'ordre de 50V).

En variante, il peut être possible de prévoir un amorçage sans haute tension, par exemple au moyen d'une électrode mobile qui viendrait en contact avec la grille pour créer l'arc et serait ensuite écartée de celle-ci une fois l'arc amorcé.

Le régime d'arc permet d'obtenir une dissociation plus importante qu'une décharge couronne, et donc assure une plus grande sensibilité au dispositif, permettant de mesurer des concentrations de l'ordre de 100 ppm ou moins. En outre, un courant élevé, outre l'augmentation de la dissociation, procure une réduction du taux d'ozone produit par la décharge. En revanche, la décharge en régime d'arc nécessite une én ergie plus importante que la décharge couronne.

Afin de neutraliser l'influence des produits oxydants tels que l'ozone, créés par une décharge électrique, on peut avantageusement utiliser deux méthodes :

. une méthode électrique, par l'utilisation d'un arc coupé qui crée un chauffage instantané détruisant l'ozone ;

. une méthode catalytique, par l'utilisation de catalyseurs destructeurs d'ozone, tels que le charbon actif.

## Revendications

1. Dispositif de détection et de mesure de concentration de gaz, du genre comportant:
   - une cellule (10) avec une conduite (11) d'entrée du gaz à analyser et une conduite (12) de sortie du gaz analysé,
   - à l'intérieur de cette cellule, des moyens (21) générateurs de haute tension propres à produire une décharge dans le gaz de manière à provoquer la dissociation du gaz,
   - en aval de la cellule des moyens détecteurs (30),
   - caractérisé en ce que pour la détection et la mesure de la concentration de $CO_2$ et CO, une seule électrode (20) est reliée auxdits moyens (21) générateurs de haute tension propre à assurer la dissociation du $CO_2$ en CO, et en ce que lesdits moyens détecteurs assurent la mesure de la concentration en CO.

2. - Dispositif selon la revendication 1, dans lequel les moyens détecteurs comprennent un capteur (30) disposé à l'intérieur de la cellule de mesure.

3. - Dispositif selon la revendication 2, dans lequel le capteur est un capteur à semi-conducteur à effet catalytique.

4. - Dispositif selon l'une des revendications précédentes, dans lequel il est prévu au moins une grille (40) d'arrêt des ions, interposée entre l'électrode et les moyens détecteurs.

5. - Dispositif selon la revendication 4, en dépendance de la revendication 2, dans lequel la grille d'arrêt est en forme de cage de Faraday entourant le capteur pour rendre celui-ci insensible aux parasites électriques ambiants.

6. - Dispositif selon la revendication 1, dans lequel les conduites d'entrée et de sortie sont réalisées en forme de venturi.

## Patentansprüche

1. Vorrichtung zur Feststellung und Messung der Gaskonzentration mit:
   - einer Zelle (10) mit einer Eintrittsleitung (11) für zu analysierendes Gas und einer Austrittsleitung (12) für analysiertes Gas,
   - einer Hochspannungsgeneratoreinrichtung (21) im Inneren dieser Zelle, zur Erzeugung einer Entladung in dem Gas zur Herbeiführung der Dissoziation des Gases,
   - einer Detektoreinrichtung (30) stromab von der Zelle,
   - dadurch gekennzeichnet, daß zum Erfassen und Messen der Konzentration von $CO_2$ und CO eine einzelne Elektrode (20) mit der genannten Hochspannungsgeneratoreinrichtung (21) verbunden ist, die geeignet ist, die Dissoziation des $CO_2$ zu CO herbeizuführen, und daß die genannte Detektoreinrichtung die Messung der CO-Konzentration vornimmt.

2. Vorrichtung nach Anspruch 1, bei welcher die Detektoreinrichtung einen im Inneren der Meßzelle befindlichen Meßfühler (30) umfaßt.

3. Vorrichtung nach Anspruch 2, bei welcher die Meßzelle ein Halbleiter-Meßwandler mit Katalysatorwirkung ist.

4. Vorrichtung nach einem der vorhergehenden Ansprüche, bei welcher zwischen der Elektrode und der Detektoreinrichtung mindestens ein Ionenfanggitter (40) vorgesehen ist.

5. Vorrichtung nach Anspruch 4, in Abhängigkeit von Anspruch 2, bei welcher das Fanggitter die Form eines Faraday-Käfigs hat, der den Meßfühler umgibt, um diesen unempfindlich gegen elektrische Störungen aus der Umgebung zu machen.

6. Vorrichtung nach Anspruch 1, bei welcher die Eintrittsleitung und die Austrittsleitung die Form von Venturirohren haben.

## Claims

1. A gas detection and concentration measuring device, of type comprising:
   - a cell (10) having an entry pipe (11) for the gas to be analyzed and an outflow (12) for the analyzed gas,
   - inside the cell, high voltage generator means (21) appropriate for producing a discharge in the gas as to bring about the dissociation of the gas,
   - downstream from the cell, detection means (30),
   - characterized in that, for the $CO_2$ and CO detection and concentration measuring, one single electrode (20) is connected to said high voltage generator means (21) appropriate for the $CO_2$ dissociation into CO, and in that said detection means provide the measuring of the CO concentration.

2. A device as claimed in claim 1, wherein the detection means comprise a sensor (30) placed inside the measuring cell.

3. A device as claimed in claim 2, wherein the sensor is a catalytic effect semiconductor sensor.

4. A device as claimed in one of the preceding claims, wherein at least one ion grid (40) is provided, placed between the electrode and the detection means.

5. A device as claimed in claim 4, when depending on claim 2, wherein the stop grid is formed as a Faraday cage surrounding the sensor in order to make the sensor insensitive to ambient electric interferences.

6. A device as claimed in claim 12, wherein the entry and outflow pipes are realized in the shape of a venturi.

FIG. 1